# EUROPEAN PATENT SPECIFICATION

(11) **EP 0 867 149 B1**
(45) Date of publication and mention of the grant of the patent: **27.09.2000**
(21) Application number: 97302163.7
(22) Date of filing: 27.03.1997
(51) Int. Cl.: A61B 17/064, A61B 17/68, A61B 17/80

(54) **Surgical bone fixation apparatus**
Chirurgische Vorrichtung zur Knochenfixierung
Dispositif d'ostéosynthèse

(43) Date of publication of application: 30.09.1998
(73) Proprietor: Jobe, Richard P., Los Altos Hills, California 94022 (US)
(72) Inventor: Jobe, Richard P., Los Altos Hills, California 94022 (US)
(74) Representative: Bayliss, Geoffrey Cyril

(56) References cited:
- EP-A- 0 230 937
- EP-A- 0 611 557
- WO-A-89/01767
- WO-A-92/17122
- WO-A-95/22930
- DE-A- 2 602 900
- NL-A- 9 201 974
- US-A- 3 939 828
- US-A- 4 655 203
- US-A- 5 167 665
- US-A- 5 290 281
- US-A- 5 443 482

## Description

This invention relates in general to a surgical bone fixation apparatus and, more particularly, to an apparatus for the fixation of bone to bone.

Various surgical plates have been employed in the treatment of traumas to facial or cranial bone structure, plastic surgery, reconstructive facial surgery, and the like to hold the bone sections or fragments in place during the healing process. The surgical plates are positioned against the surface of the bone sections which must be held together and fixed to the bone by mechanical fasteners such as bone screws, wire sutures or other fasteners which secure the plate to the bone surface. The fasteners are securely pressed or embedded in the bone to prevent the surgical plates from being pulled away from the surface of the bone sections. U.S. Patents 4,966,599 and 5,290,281 disclose examples of bone stabilization plates which are secured to the bone structure, for example facial or cranial bones, by bone screws. U.S. Patent 4,655,203 discloses a surgical device for immobilization of bone fracture which includes a stiff plate and stiff fixing elements which may be pressed into notches formed in the bone.

In such procedures as rotator cuff surgery and hand tendon surgery, tendons or other soft tissues are fixedly secured to bone. The tendons or other tissue are often secured to bone by feeding the soil tissue through holes formed in the bone and suturing the tissue in place. Another method of securing soft tissue to bone employs a fixation device which essentially tacks the tendon to bone. Unless the tendon or other soft tissue is completely immobilized, the fixation device must be securely embedded in the bone to hold the tendon stationary against the bone surface, resisting those forces tending to pull the tendon away from the bone surface, until the tendon has become attached to the bone. Another method of securing tendons and other soft tissue to bone uses a fixation device with an attached suture. Once again, the fixation device must be securely embedded in bone to resist forces tending to pull the tissue away from the bone surface. In various facial surgery procedures, soft tissue is surgically lifted or moved and then secured to bone by suturing and the like to hold the tissue in the desired position during the healing process. These tissues are generally subjected only to gravitational forces or other forces parallel to the surface of the bone, they are not exposed to forces tending to pull the tissue outwardly away from the bone.

Surgical plates must be securely affixed to bones which may be exposed to various tensile and bending forces during the healing process to prevent the plate from being pulled outwardly away from the bone. If the fasteners do not securely engage the bone, the applied stresses may have a tendency to pull the fasteners from the bone. Facial and cranial bone sections, on the other hand, are generally not exposed to such tensile and bending stresses. Instead, the bone sections are primarily subjected to forces tending to spread the bone sections apart or to cause lateral slippage of the bone sections along the fracture line. Since the applied forces are substantially parallel to the bone surface, the fasteners need only anchor the surgical plates to the facial or cranial bones to hold the bone sections in place. The additional security provided by securely embedding the fastener in bone and actually securing the plate to the bone surface is often not required with facial and cranial bones and of bones at other sites.

Securely embedding screws, nails and the like in bone is a time consuming and labor intensive process, considerably extending the time required to complete the operation. Even when the fasteners are initially inserted into pre-drilled holes, care must be taken to ensure that the desired orientation of the fasteners is maintained. Moreover, embedding the fasteners in bone may subject the relatively fragile facial bones to additional unnecessary stress. Surgical fasteners such as screws and the like require expensive manufacturing techniques because of the small size of the fastener. Minimizing the size of the plate and fastener would minimize the size of the surgical area and the amount of bone which must be exposed. However, the reduced size of the screws and other fasteners is limited because the fasteners have sufficient strength to engage bone and securely attach the plate to the bone. Using a fixation device which anchors the surgical plate to the bone, but does not securely affix the plate to the bone surface, would avoid the disadvantages associated with embedding the fastener in bone. Such a fixation device would also be particularly useful in other types of procedures where the fixation apparatus may be employed to affix soft tissue to bone.

The surgical plates and associated fasteners employed for bone fixation have been fabricated of materials such as titanium, stainless steel, vitalium, chrome cobalt, and suitable bio-compatible polymeric materials. Unless removed by surgery, the plates and fasteners formed of these materials permanently remain in the patient's body. The surgical plates and internal fastening members may cause various unpredictable problems if left intact as the bone dynamically reacts to the foreign bodies over time by molding to the shape of the foreign body, forming deposits on the foreign body, and responding to stress. The foreign bodies also provide potential cites for infection. Migration of the foreign body presents another problem if the surgical plate and/or fasteners are left in the patient's body. This problem is of particular concern when the patient is an infant or young child, where the considerable amount of skull growth has resulted in significant migration of foreign body to the extent where the plate has entered the brain.

Forming the plate and/or fasteners of a material which may be absorbed by the body over time would allow the foreign materials to be removed from the patient's body without requiring a second operation. U.S. Patents 4,655,203, 4,905,680, 4,966,599, 5,275,601 and 5,290,281 describe forming the surgical plates and/or fasteners of absorbable materials. Fabricating a bone screw or other mechanical fastener out of absorbable polymers is often difficult because the absorbable fastener has a tendency to prematurely slip from the bone. Moreover, because of their reduced size, forming absorbable bone screws for the fixation of plates to facial and cranial bones is even more difficult. To achieve the desired strength, the absorbable screws must be larger in size than a comparable metal screw. A fixation device for the fixation of surgical plates to bone which may be easily manufactured of an absorbable material and reliably used is highly desirable.

US-A-3,939,828 describes a clasp formed of spring material that is Z-shaped in top plan and in side elevation shows two legs which converge downwardly at about a 15 degree angle, one leg depending from each end of the Z. To install the clasp, bone segments are manually or mechanically manipulated to the desired position. A hole is drilled on each side of the osteotomy equidistant therefrom at an angle of about 15 degrees from the perpendicular, and converging toward the osteotomy preferably using a template which determines hole locations, dependent on the size of clasp selected. One leg of the clasp is inserted in one hole. The other leg is pulled toward the other hole, preferably using an instrument with a hook-like extremity. As the second leg is pulled into position the clasp is stressed, the Z shape spreading apart; and after the clasp is fully installed, the resiliency of the clasp applies a continuous force compressing the bone segments with a constant force and immobilizing the same.

WO-A-92/17122 describes an essentially U-shaped binding clamp made of a solid material and composed of a base fixed to two drive-in branches. The binding clamp is deformed before being driven in a deformed state into a material by means of a driving-in device.

NL-A-9201974, on which the two part form of claim 1 is based, describes slidably insertable posts.

It is a primary object of this invention to provide a bone fixation apparatus for the surgical fixation of bone or soft tissue to bone.

It is another object of this invention to provide a bone fixation apparatus for anchoring a surgical plate, suturing element or other surgical member to bone.

It is yet another object of this invention to provide a bone fixation apparatus which may be quickly and easily applied to bone by the surgeon without subjecting the bone to unnecessary stresses.

It is still another object of this invention to provide a bone fixation apparatus in which the size of the post fixation device may be minimized.

It is a further object of this invention to provide a bone fixation apparatus which may be fabricated of materials which are absorbed by the body over time.

A more general object of this invention is to provide a bone fixation apparatus which may be economically manufactured and which may be efficiently and reliably employed in the fixation of surgical plates, suturing elements and other surgical members to bone.

According to the invention there is provided a bone fixation apparatus according to Claim 1.

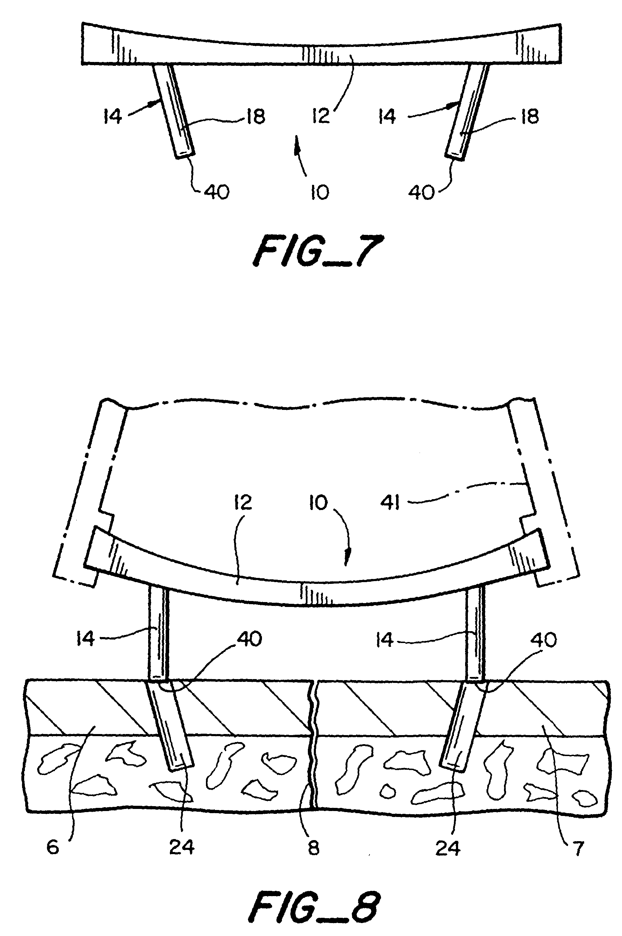

Additional objects and features of the invention will be more readily apparent from the following detailed description and appended claims when taken in conjunction with the drawings, of which only figures 7 and 8 depict an embodiment of the invention as claimed.

Figure 1 is an enlarged, fragmentary, cross sectional view of a bone fixation apparatus, shown fixed to two bone sections.

Figure 1A is an enlarged, fragmentary view of a modified post device.

Figure 2 is an enlarged, fragmentary, top plan view of the bone fixation apparatus and bone sections of Figure 1.

Figure 3 is an enlarged, fragmentary, top plan view of a bone fixation apparatus.

Figure 4 is an enlarged, fragmentary, top plan view of a bone fixation apparatus.

Figure 5 is a fragmentary, cross sectional view taken along line 5-5 of Fig 4.

Figures 6 and 6A are schematic, top plan views of a bone fixation apparatus.

Figure 7 is an enlarged, side elevational view of a bone fixation apparatus in accordance with the invention.

Figure 8 is an enlarged, side elevational view of the bone fixation apparatus of Figure 7, shown during application of the apparatus to the bone sections.

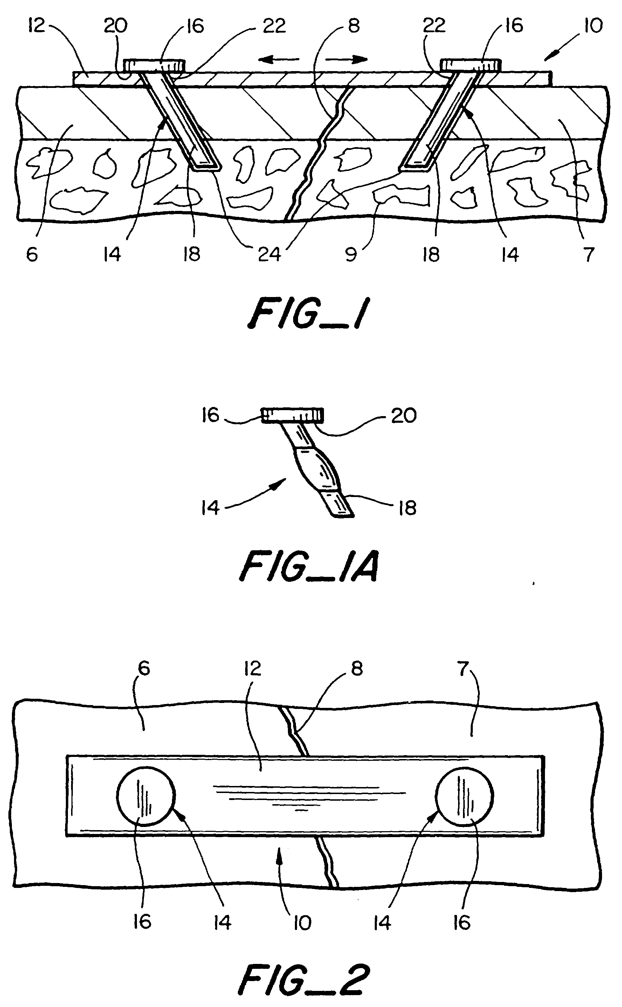

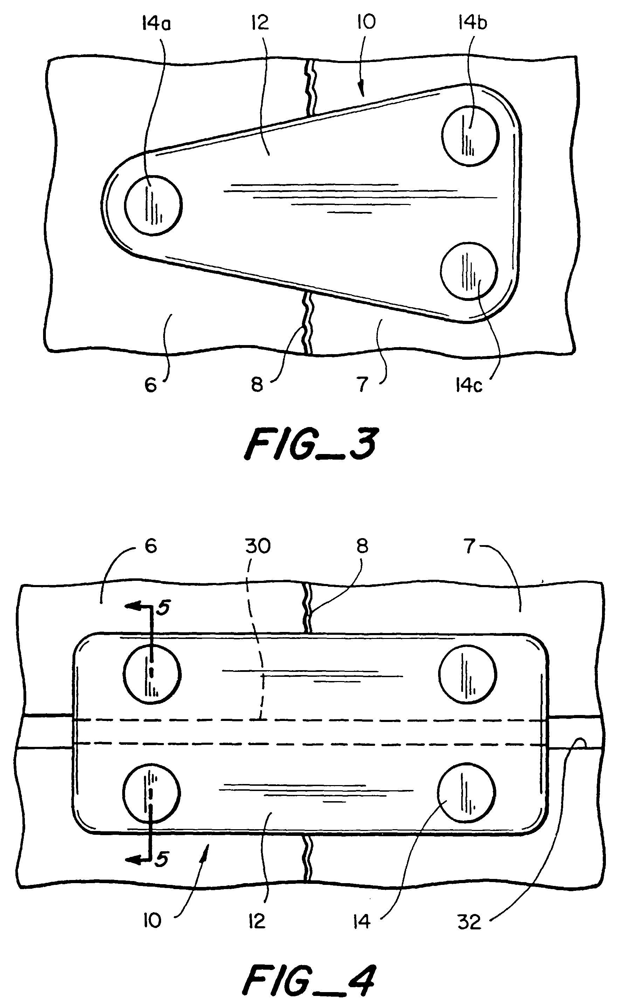

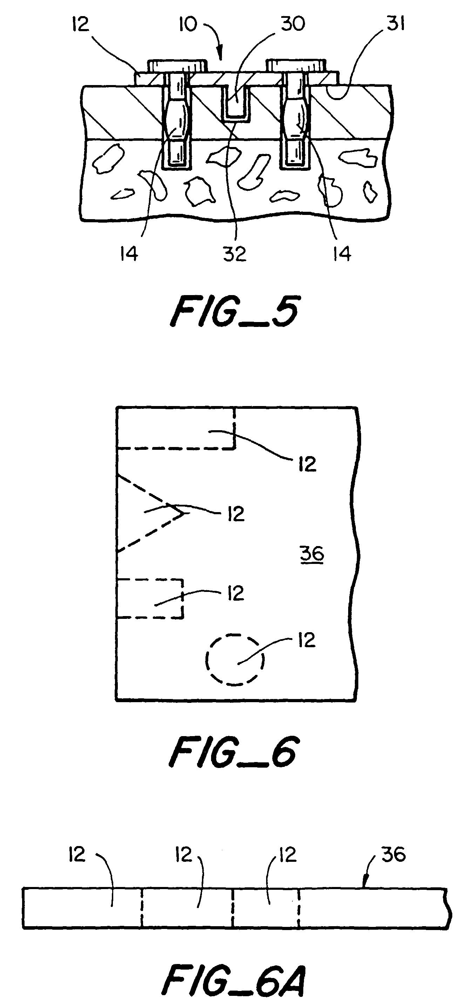

Reference will now be made in detail to the accompanying figures. Turning now to the drawings, wherein like components are designated by like reference numbers throughout the various figures, attention is directed to Figures 1 and 2.

A bone fixation apparatus 10 may be used for the fixation of bone or soft tissue to bone. In the modification of Figures 1 and 2, the bone fixation apparatus 10 is shown holding two bone sections 6 and 7 in a desired position about a fracture line or incision, generally designated 8. Fixation apparatus 10 may be used with any type of bone structure as for example cancellous bones with marrow 9, bones without marrow such as the walls of the sinus, and the skull which includes an outer table, an inner table and marrow between the inner and outer tables. Preferably, the fixation apparatus 10 is used with bones which are subjected primarily to forces parallel to the bone surface, such as those which tend to separate the bone sections or cause relative lateral movement of the bone sections. The bones are generally isolated from forces transverse to the bone surface and are not required to carry a significant load. Examples of such bones include the facial and cranial bones, although it will be understood that use of the bone fixation apparatus may also be used with other suitable bones.

In the device shown in Figure 1, bone fixation apparatus 10 includes a surgical plate member 12 extending across the surface of the bone and post devices 14 retaining plate member 12 in place. The shape, size and thickness of plate member 12 is subject to considerable modification depending upon the location of bone sections 6 and 7, the size of fracture line 8, the characteristics of the material of plate member 12, the characteristics and desired result of the surgical procedure employing the apparatus and the like. Surgical plate member 12 is anchored to each bone section by at least one post device 14. In many applications, using one post device 14 per bone section as shown in Figures 1 and 2 will sufficiently retain the bone sections in the desired position. However, using more than one post device for at least one of the bone sections offers the advantage of increased stability and augmented parallelographic force. For example, Figure 3 shows a modification where one post device 14a anchors plate member 12 to bone section 6 and two post devices 14b and 14c anchor the plate to bone section 7. The positioning of post devices 14a, 14b and 14c creates a triangulation effect which substantially resists relative lateral movement of the bone sections along the fracture line. Instead of using three post devices 14 for enhanced stability as shown in Figure 3, a greater number of post devices 14 may be employed if desired.

If additional security is desired, plate member 12 may be secured to bone by replacing one of the post devices 14, for example post device 14a shown in Figure 3, with a screw or other suitable fastener which securely engages the walls of hole 24 to attach the plate member to the surface of the bone section. The remaining post devices, such as post devices 14b and 14c, anchor the plate member 12 to the other bone section to retain the bone sections in the desired position.

As shown in Figure 1, post device 14 includes an enlarged cap or end portion 16 and a leg portion 18 depending from the underside 20 of end portion 16. The leg portion 18 extends through an aperture 22 formed in plate member 12 and is positioned in a hole 24 formed in bone sections 6 and 7 to anchor surgical plate 12 to the bone sections. Unlike the fixation devices of the prior art, post device 14 is not intended to prevent the surgical plate 12 from being pulled away from the surface of the bone sections. Instead, post device 14 substantially resists forces parallel to the surface of the bone, such as those tending to separate the bone sections or cause lateral slippage along the fracture line, to hold surgical plate 12 in place. Leg portion 18 is formed of sufficient length relative to the interior diameter of the hole 24 to resist those forces parallel to the bone surface. The optimum length for leg portion 18 depends in part on such factors as the stiffness of the material used in the fabrication of post device 14 and the magnitude of the forces applied to the post device. For many applications, a length of at least about two times the interior diameter of the hole 24 will be sufficient to prevent forces parallel to the bone surface from deforming the leg portion and pulling the leg portion 18 from the bone. However, it will be understood that in some applications it may be desirable to provide leg portion 18 with a lesser or greater length or angle.

Since it is the length of the leg portion 18 which holds the post device 14 in the hole and anchors the plate member 12 to the bone sections, leg portion 18 may be shaped so that only a minimum amount of pressure is required to slip the leg portion into the hole 24. The leg portion 18 is not securely embedded in place, but is instead movable in a direction parallel to the axis of the hole. The exterior of leg portion 18 may be substantially cylindrical as shown in Figure 1, or, if desired, leg portion 18 may be configured to provide a limited area of engagement between the exterior of the leg portion and the walls of hole 24. For example, Figure 1A shows a modification of post device 14 having a slightly bowed or spindle-shaped cross section, although it is to be understood that leg portion 18 may have other cross sectional shapes if desired. Moreover, the area of limited engagement need not extend around the entire circumference of the leg portion 18. Instead, leg portion 18 may be formed with one or more projections which are configured to slightly engage the interior wall of hole 24.

The maximum diameter of the spindle-shaped leg portion shown in Figure 1A is preferably equal to or slightly greater than the interior diameter of the hole 24 so that when the post device 14 is initially inserted in the hole, the leg portion will partially engage the wall of the hole. Although the limited engagement between the leg portion and wall of the bone is generally insufficient to securely retain the post in the hole 24, the spindle shape of leg portion 18 allows the surgeon to conveniently manipulate the surgical plate relative to the bone sections during the surgical procedure without pulling the post device from the hole 24. Since only a limited area of leg portion 18 engages the walls of the bone, the pressure required to insert the post device 14 into hole 24 is not significantly increased.

As is shown particularly in Figure 1, leg portion 18 is preferably oriented at an angle of about 15 to 55 degrees relative to the underside 20 of the end portion 16. For optimum effectiveness, the angled leg portion 18 is inserted into a hole 24 extending into the bone at approximately the same angle as the inclination of leg portion 18 so that the underside 20 of the end portion engages the upper surface of the plate member 12. Aperture 22 may also extend through plate member 12 at an angle. When post device 14 is inserted in the bone, the angled leg portion 18 is preferably oriented so that the tip of the leg portion points in a direction opposite the forces applied to the bone section. For example, in Figure 1 the angled leg portion 18 is oriented inwardly toward the fracture line, extending in a direction opposite those forces tending to pull the bone sections 6 and 7 apart.

Providing post device 14 with a slanted leg portion increases the stability of bone fixation apparatus 10 in resisting forces parallel to the bone sections 6 and 7. When positioned as shown in Figure 1, the slanted leg portions of the post devices 14 on opposite sides of the fracture line may also be used to urge the bone sections 6 and 7 together. Urging the bone sections together with post devices 14 ensures the bone sections are held together, facilitating the healing process. Although angled leg portions 18 increase the ability of post device 14 to resist forces substantially parallel to the bone sections, it will be understood that the leg portions 18 may also be perpendicular to the underside of end portion 16 if desired.

The bone fixation apparatus 10 may be applied to the selected bone sections using a suitable surgical technique. Apertures 22 may be formed in plate member 12 at predetermined locations or, if desired, the surgeon may select the site of the apertures. If desired, the apertures 22 and holes 24 may be formed simultaneously by locating the plate member 12 on the bone sections 6 and 7 and drilling through the plate and bone. However, forming the apertures 22 separately from the holes 24 isolates the wound or surgical site from unwanted surgical plate fragments. The locations of holes 24 may be selected using a template if desired. Preferably, holes 24 are formed using a drill or other appropriate instrument having a stop or collar limiting the hole depth to avoid excess penetration particularly in the skull. When post devices 14 having angled leg portions 18 are employed, the stop is preferably oriented at an angle relative to the drill bit similar to the angle between the end portion 16 and leg portion 18. Alternatively, the stop may be curved for usage with post devices angled or perpendicular to the end portion 16.

The configuration of surgical plate member 12 is subject to considerable variation depending upon the constraints of a particular application. Figure 2 shows a plate member 12 having a substantially rectangular shape. The plate member may also have other shapes such as the triangular shape shown in Figure 3. Figures 4 and 5 show another device in which plate member 12 includes a flange or rib 30 depending from the underside 31 of the plate member 12. In the device shown in Figures 4 and 5, the flange 30 extends continuously along the entire length of the plate member. However, flange member 30 may have other configurations if desired. Flange 30 is positioned in a thin groove or cut 32 formed in the bone to assist in obtaining the desired positioning of bone fixation apparatus 10 and bone sections 6 and 7 when the apparatus 10 is initially anchored to the bone. The inter-engagement of flange 30 and groove 32 also provides additional resistance against relative lateral slippage between the bone sections 6 and 7. In the modification shown in Figures 4 and 5, four post devices 14 having a spindle-shaped cross section are employed to anchor plate member 12 to bone. The post devices 14 may have slanted leg portions or leg portions substantially perpendicular to the plate member 12.

Although not shown, the plate member may also be formed with a curved or stepped cross-section. The curved or stepped configuration may be prefabricated or the plate member may be formed of a suitable material and manipulated into the desired shape by the surgeon or surgical assistant. The prefabricated plate member may also be formed of a material which allows the shape of the plate member to be adjusted during the operation.

Figure 6 illustrates a modification in which plate member 12 comprises a section of an elongated sheet 36 of surgical plate material. The sheet 36 shown in Figure 6 is of sufficient width to allow the surgeon select any shape desired for the surgical plate. Alternatively, as is shown in Figure 6A the sheet 36 may be formed as a narrow strip of material which may be separated into plate sections of arbitrary lengths. The ability to divide the sheet 36 of plate material into plate sections each having an arbitrary shape provides the surgeon with immediate access to a plate member 12 of appropriate size. The sheet 36 of surgical plate material may be divided into separate plate sections using the appropriate clipping tools for the particular material of sheet 36. Depending upon the material employed for surgical sheet 36, the sheet 36 may be retained in a roll or provided as a planar sheet of material. As with the previously described devices, apertures may be pre-formed in sheet 36 or the apertures may be formed by the surgeon at the desired locations. Alternatively, as will be described in greater detail in relation to Figures 7 and 8, sheet 36 may be integrally formed with a plurality of spaced post devices 14.

Turning to Figures 7 and 8, bone fixation device 10 is a monolithic structure in which post devices 14 are integrally formed with plate member 12. In the device according to the invention as shown in Figures 7 and 8, the leg portions 18 of the post devices 14 are oriented at an angle relative to the underside of plate member 12. The fixation apparatus is applied to bone by deforming the edges of plate member 12 upwardly as shown in Figure S to position the tips 40 of the post device above the open ends of the holes 24. Preferably, the upper surface of the plate 12 is concave to facilitate deformation of the plate to bring the post devices 14 into a substantially parallel orientation. The post devices 14 are then slipped into the holes and the plate member 12 is moved toward the bone surface. The plate member 12 returns to its original shape shown in Figure 7, with the angled post devices 14 providing addition resistance against separation, when the outer edges of the plate member are released and the plate member is positioned against the bone surface. Preferably, a surgical instrument 41 is used to bend the plate 14 and hold the plate as shown in Figure 8, allowing a nurse or surgical technician to prepare the plate for insertion and allowing the surgeon to manipulate the plate to the desired position. The surgical instrument may also be used to adjust the position of the fixation apparatus or to remove the fixation apparatus from the bone.

The post devices 14 anchor the fixation apparatus to bone to retain the bone sections in the desired position during healing. As previously described, the angled leg portions 18 provide increased resistance to oppose forces substantially parallel to the bone surface and retain the bone sections in place. In the present embodiment, the post devices are preferably oriented at an angle of approximately 10-20 degrees to minimize the amount of deformation of plate 12 which is required to insert the post devices 14 into holes 40.

Although slanted leg portions 18 are preferred for increased stability, post devices 14 may also extend in a direction substantially perpendicular to the underside of the plate member 12. With the perpendicular post devices 14, upward deformation may not be required to align the tips 40 of the post devices with the holes 24 formed in the bone sections.

As is apparent from the foregoing description, the bone fixation apparatus is particularly suitable for holding bone sections in the desired position. The post devices anchor the surgical member, such as the surgical plate to bone without securely engaging the bone and attaching the surgical member to the bone surface. The size of the post device may be minimized. For example, the post device may have a leg diameter on the order of about 1 mm The bone fixation apparatus may be formed of any suitable bio-compatible or absorbable materials. Examples of suitable materials include, but are not limited to, bio-compatible metals, bio-compatible elastomers exhibiting sufficient stiffness properties and other bio-compatible polymers, and bio-absorbable polymers which are partially or completely absorbed by the body after time may also be used.

## Claims

1. A bone fixation apparatus (10) for retaining adjacent bone sections (6) and (7) in a desired position during the healing process, said bone sections (6) and (7) each having an outer surface and at least one hole (24) formed therein, said hole having an interior diameter, said bone fixation apparatus (10) comprising a plate member (12) configured to extend across a portion of each of said bone sections (6) and (7), said plate member (12) having an upper surface and a lower surface positionable on said outer surface of said bone sections (6) and (7), and at least one post device (14) carried by said plate member (12) for each of said bone sections (6) and (7), each said post device (14) having a leg portion (18) configured for slidable insertion into said hole (24), said leg portion (18) being of sufficient length relative to said interior diameter of said hole (24) to resist removal of said leg portion (18) from said hole (24) when forces substantially parallel to said outer surface of said bone sections (6) and (7) are applied to said post device (14) to anchor said plate member (12) to said bone sections (6) and (7) and retain said bone sections (6) and (7) in said desired position, characterised in that said leg portion (18) of each said post device (14) is oriented at an angle relative to said lower surface of said plate member (12), and in that said plate member (12) is resiliently deformable from a first undeformed position to a second position suitable for insertion of said leg portion (18) of each said post device (14) into a respective one of said holes (24), said holes having been arranged to conform to the relative positions of said leg portions of said post devices with the plate member in its first position.

2. The bone fixation apparatus (10) of Claim 1 in which said leg portion (18) has an enlarged portion having a maximum diameter greater than said interior diameter of said hole (24), said enlarged portion being shaped to engage said bone when said leg portion (18) is inserted in said hole (24).

3. The bone fixation apparatus (10) of Claim 1 in which said plate member (12) has a plurality of apertures formed therein, each said post device (14) being positioned in one of said apertures with said leg portion (18) of said post device (14) depending from said lower surface of said plate member (12).

4. The bone fixation apparatus (10) of Claim 1 in which said post device (14) includes an enlarged end portion shaped to engage a portion of said upper surface of said plate member (12) adjacent said one of said apertures.

5. The bone fixation apparatus (10) of Claim 1 in which said leg portion (18) of said post device (14) is inclined at an angle of about 10 to 20 degrees relative to said lower surface of said plate member (12).

6. In combination, the bone fixation apparatus (10) of Claim 5 and an instrument (41) engaging said plate member (12) and retaining said plate member (12) in said second position.

7. The bone fixation apparatus (10) of Claim 1, and further comprising a fastener (14a) mountable to said plate member (12) and one of said bone sections (6) and (7) to secure said plate member (12) to said one of said bone sections (6) and (7), said fastener (14a) having a longitudinal axis, said fastener (14a) being configured to substantially resist removal of said fastener (14a) from said bone upon application of a force substantially parallel to said longitudinal axis.

8. The bone fixation apparatus (10) of Claim 1 in which at least one of said plate member (12) and said post device (14) are formed of an absorbable material.

## Patentansprüche

1. Knochenfixiervorrichtung (10) zum Halten benachbarter Knochenabschnitte (6) und (7) in einer gewünschten Position während des Heilprozesses, wobei
- die Knochenabschnitte (6) und (7) jeweils eine Außenfläche und wenigstens ein darin ausgebildetes Loch (24) aufweisen,
- das Loch einen Innenduchmesser hat,
- die Knochenfixiervorrichtung (10) ein Plattenelement (12) aufweist, das so gestaltet ist, daß es sich über einen Teil eines jeden der Knochenabschnitte (6) und (7) erstreckt,
- das Plattenelement (12) eine obere Fläche und eine untere an der Außenfläche der Knochenabschnitte (6) und (7) positionierbare Fläche, und wenigstens eine Stangeneinrichtung (14) für jeden der Knochenabschnitte (6) und (7) hat, die von dem Plattenelement (12) getragen wird,
- jede Stangeneinrichtung (14) einen Schenkelabschnitt (18) hat, der für ein Schiebe-Einführen in das Loch (24) ausgebildet ist,
- der Schenkelabschnitt (18) bezüglich des Innendurchmessers des Lochs (24) eine ausreichende Länge hat, um einem Entfernen des Schenkelabschnitts (18) aus dem Loch (24) zu widerstehen, wenn zur äußeren Fläche der Knochenabschnitte (6) und (7) im wesentlichen parallele Kräfte an die Stangeneinrichtung (14) angelegt werden, wodurch das Plattenelement (12) an den Knochenabschnitten (6) und (7) verankert und die Knochenabschnitte (6) und (7) in der gewünschten Position gehalten werden,
dadurch gekennzeichnet,
- daß der Schenkelabschnitt (18) einer jeden Stangeneinrichtung (14) in einem Winkel bezüglich der unteren Fläche des Plattenelements (12) ausgerichtet ist, und
- daß das Plattenelement (12) aus einer ersten unverformten Position in eine zweite Position elastisch verformbar ist, die für ein Einführen des Schenkelabschnitts (18) einer jeden Stangeneinrichtung (14) in das entsprechende Loch (24) geeignet ist,
- wobei die Löcher so angeordnet sind, daß sie mit den entsprechenden Positionen der Schenkelabschnitte der Stangeneinrichtungen übereinstimmen, wenn sich das Plattenelement in seiner ersten Position befindet.

2. Knochenfixiervorrichtung (10) nach Anspruch 1, bei welcher der Schenkelabschnitt (18) einen vergrößerten Teil mit einem maximalen Durchmesser hat, der größer ist als der Innendurchmesser des Lochs (24), wobei der vergrößerte Teil so geformt ist, daß er an dem Knochen angreift, wenn der Schenkelabschnitt (18) in das Loch (24) eingeführt ist.

3. Knochenfixiervorrichtung (10) nach Anspruch 1, bei welcher das Plattenelement (12) eine Vielzahl von in ihm ausgebildeten Öffnungen hat, wobei jede Stangeneinrichtung (14) in einer der Öffnungen positioniert ist und der Schenkelabschnitt (18) der Stangeneinrichtung (14) von der unteren Fläche des Plattenelements (14) weg aufgehängt ist.

4. Knochenfixiervorrichtung (10) nach Anspruch 1, bei welcher die Stangeneinrichtung (14) einen vergrößerten Stirnabschnitt hat, der so geformt ist, daß er an einem Teil der oberen Fläche des Plattenelements (12) angrenzend an eine der Öffnungen angreift.

5. Knochenfixiervorrichtung (10) nach Anspruch 1, bei welcher der Schenkelabschnitt (18) der Stangeneinrichtung (14) in einem Winkel von etwa 10 bis 20° bezüglich der unteren Fläche des Plattenelements (12) geneigt ist.

6. Kombination der Knochenfixiervorrichtung (10) nach Anspruch 5 mit einem Werkzeug (41), das an dem Plattenelement (12) angreift und das Plattenelement (12) in der zweiten Position hält.

7. Knochenfixiervorrichtung (10) nach Anspruch 1, welche weiterhin eine Befestigungseinrichtung (14a) aufweist, die an dem Plattenelement (12) und an einem der Knochenabschnitte (6) und (7) anbringbar ist, um das Plattenelement (12) an einem der Knochenabschnitte (6) und (7) festzulegen, wobei die Befestigungseinrichtung (14a) eine Längsachse hat und so gestaltet ist, daß sie einem Entfernen der Befestigungseinrichtung (14a) aus dem Knochen bei Anlegen einer Kraft im wesentlichen parallel zur Längsachse im wesentlichen widersteht.

8. Knochenfixiervorrichtung (10) nach Anspruch 1, bei welcher wenigstens das Plattenelement (12) oder wenigstens die Stangeneinrichtung (14) aus einem absorbierbaren Material hergestellt ist.

## Revendications

1. Appareil (10) de fixation d'os destiné à retenir des sections d'os adjacentes (6) et (7) dans une position souhaitée pendant le processus de consolidation, lesdites sections d'os (6) et (7) ayant chacune une surface extérieure et au moins un trou (24) formé en elles, ledit trou ayant un diamètre intérieur, ledit appareil (10) de fixation d'os comportant un élément à plaque (12) configuré de façon à s'étendre sur une partie de chacune desdites sections d'os (6) et (7), ledit élément à plaque (12) ayant une surface supérieure et une surface inférieure pouvant être positionnée sur ladite surface extérieure desdites sections d'os (6) et (7), et au moins un dispositif à broche (14) porté par ledit élément à plaque (12) pour chacune desdites sections d'os (6) et (7), chacun desdits dispositifs à broche (14) ayant une partie formant tige (18) configurée pour être insérée en coulissant dans ledit trou (24), ladite partie formant tige (18) étant d'une longueur suffisante par rapport audit diamètre intérieur dudit trou (24) pour résister à l'enlèvement de ladite partie formant tige (18) dudit trou (24) lorsque des forces sensiblement parallèles à ladite surface extérieure desdites sections d'os (6) et (7) sont appliquées audit dispositif à broche (14) pour ancrer ledit élément à plaque (12) auxdites sections d'os (6) et (7) et retenir lesdites sections d'os (6) et (7) dans ladite position souhaitée, caractérisé en ce que ladite partie formant tige (18) de chacun desdits dispositifs à broche (14) est orientée d'un certain angle par rapport à ladite surface inférieure dudit élément à plaque (12), et en ce que ledit élément à plaque (12) peut être déformé élastiquement d'une première position non déformée à une seconde position convenant à l'introduction de ladite partie formant tige (18) de chacun desdits dispositifs à broche (14) dans l'un respectif desdits trous (24), lesdits trous étant agencés pour se conformer aux positions relatives desdites parties formant tige desdits dispositifs à broche avec ledit élément à plaque dans sa première position.

2. Appareil de fixation d'os (10) selon la revendication 1, dans lequel ladite partie formant tige (18) comporte une partie élargie ayant un diamètre maximal plus grand que ledit diamètre intérieur dudit trou (24), ladite partie élargie étant configurée de façon à entrer en prise avec ledit os lorsque ladite partie formant tige (18) est introduite dans ledit trou (24).

3. Appareil (10) de fixation d'os selon la revendication 1, dans lequel ledit élément à plaque (12) présente plusieurs ouvertures formées en lui, chacun desdits dispositifs à broche (14) étant positionné dans l'une desdites ouvertures de façon que ladite partie formant tige (18) dudit dispositif à broche (14) fasse saillie vers le bas de ladite surface inférieure dudit élément à plaque (12).

4. Appareil (10) de fixation d'os selon la revendication 1, dans lequel ledit dispositif à broche (14) comprend une partie extrême élargie configurée pour porter contre une partie de ladite surface supérieure dudit élément à plaque (12) à proximité immédiate de ladite, une, desdites ouvertures.

5. Appareil (10) de fixation d'os selon la revendication 1, dans lequel ladite partie formant tige (18) dudit dispositif à broche (14) est inclinée d'un angle d'environ 10 à 20° par rapport à ladite surface inférieure dudit élément à plaque (12).

6. En combinaison, l'appareil (10) de fixation d'os selon la revendication 5 et un instrument (41) prenant ledit élément à plaque (12) et retenant ledit élément à plaque (12) dans ladite seconde position.

7. Appareil (10) de fixation d'os selon la revendication 1, comportant en outre un organe d'assujettissement (14a) pouvant être monté sur ledit élément à plaque (12) et l'une desdites sections d'os (6) et (7) pour assujettir ledit élément à plaque (12) à ladite une desdites sections d'os (6) et (7), ledit organe d'assujettissement (14a) ayant un axe longitudinal, ledit organe d'assujettissement (14a) étant configuré pour résister sensiblement à l'enlèvement dudit organe d'assujettissement (14a) dudit os lors de l'application d'une force sensiblement parallèle audit axe longitudinal.

8. Appareil (10) de fixation d'os selon la revendication 1, dans lequel au moins l'un dudit élément à plaque (12) et dudit dispositif à broche (14) est formé d'une matière absorbable.
